# EUROPEAN PATENT SPECIFICATION

(11) **EP 1 740 508 B1**
(45) Date of publication and mention of the grant of the patent: **05.12.2018**
(21) Application number: 05739010.6
(22) Date of filing: 28.04.2005
(51) Int. Cl.: C02F 11/04

(54) **METHOD AND APPARATUS FOR TREATING ORGANIC DRAINAGE AND SLUDGE**
VERFAHREN UND VORRICHTUNG ZUR BEHANDLUNG VON ORGANISCHER DRÄNAGE UND SCHLAMM
PROCÉDÉ ET APPAREIL POUR LE TRAITEMENT DE DRAINAGE ORGANIQUE ET DE BOUE

(30) Priority: 28.04.2004 JP 2004133635
(43) Date of publication of application: 10.01.2007
(73) Proprietor: Ebara Engineering Service Co., Ltd., Tokyo 144-8610 (JP)
(72) Inventor: HAGINO, Takao, Ohta-ku, Tokyo 1448510 (JP); KOBAYASHI, Atsushi, Ohta-ku, Tokyo 1448510 (JP); NOMURA, Junichi, Ohta-ku, Tokyo 1448510 (JP); SAWAI, Kenji, Ohta-ku, Tokyo 1448510 (JP); ISHIKAWA, Hideyuki, Ohta-ku, Tokyo 1448510 (JP); HIRAJIMA, Tsuyoshi, Ohta-ku, Tokyo 1448510 (JP)
(74) Representative: Wagner & Geyer
(86) International application number: PCT/JP2005/008547
(87) International publication number: WO 2005/105680

(56) References cited:
- DE-C1- 19 532 802

## Description

### TECHNICAL FIELD

The present invention relates to a system of treating organic drainage in sewage treatment facilities and various types of waste water treatment facilities, and more specifically, it relates to a technique of separating and removing fine particles such as heavy metal compounds from drainage containing high concentration organic substances, phosphorus and nitrogen and a small amount of a heavy metal component, and furthermore it relates to a sludge treatment method and apparatus for efficiently separating and recovering useful resources such as magnesium ammonium phosphate (hereinafter referred to as "MAP") crystals existing in organic sludge and the like.

### BACKGROUND ART

The conventional methods of treating heavy metals from organic sludge (hereinafter referred to simply as "sludge") include, for example, a method of adding an acid to sludge, a dried product of sludge or ashes obtained by incinerating the sludge, to elute heavy metal ions, and then (1) adding an alkali to the obtained eluate, and thereafter adding ferric chloride and a polymeric flocculant to perform flocculation and precipitation treatment, (2) subjecting the eluate to solid-liquid separation and thereafter removing heavy metal ions by an ion-exchange method, or (3) subjecting the eluate to electrodeposition by an electrolysis treatment using a Cu electrode and Cd electrode. All these methods have problems such that a large amount of the acid should be added to the sludge or the like in the pretreatment and the running cost is very high due to addition of chemicals such as an pH adjustor and an oxidizing agent, use of expensive ion exchange resin, or electric power for electrolysis, in the subsequent treatment, and furthermore they are mostly complicated as treating systems. Besides, various substances other than heavy metals are generally mixed in sludge, and this has caused a lowering of heavy metal removal performance. Taking sewer sludge formed in a sewage treatment facilities as an example, in addition to heavy metals, a large amount of various substances including, for example, organic substances derived from microorganisms, silt, hairs, plant or fruit seeds and soluble components such as carbonic acid, ammonia and phosphoric acid are mixed in the sludge. Thus, there has been a problem of very low efficiency of the system of collectively treating sludge substances including these other substances in order to remove very slight amount of harmful heavy metals contained in the sludge substances.

Then, in order to solve the above described problems, the present inventors has enabled selective separation and removal of heavy metal derived substances in the form of particulate substances as such existing in the sludge or in the form of the particles having specific characteristics obtained by modifying the heavy metal derived substances using a relatively easily modifying method by a fine particle separation system. Furthermore, the inventors have enabled simultaneous separation and recovery of other various particles to be recovered as valuable substances in the sludge by taking separation means in accordance with respective particle properties. Further, the present invention utilizes the technique of efficiently recovering the phosphorus in waste water as MAP by "Method and Apparatus for Treating Organic Wastewater" disclosed in Japanese Patent Application Nos. 2000-231633 (JP-A1-2002-45889), 2002-186179, 2002-116257 and the like (hereinafter referred to by the number alone).

### DISCLOSURE OF THE INVENTION

### PROBLEMS TO BE SOLVED BY THE INVENTION

The present invention solves the above described problems and has an object to further improve the system disclosed in Japanese Patent Application Nos. 2000-231633, 2002-186179, 2002-116257 and the like as mentioned above. That is, the present invention has an object to provide a method and apparatus for treating sludge which enable formation of sludge having a comparatively low heavy metal content ratio and recovery of useful valuable substances as well with ease and at a low cost, by separating and removing heavy metal derived particles from organic drainage or sludge containing high concentration organic substances, phosphorus and nitrogen and efficiently recovering valuable particles such as MAP. Further, in the present invention, the organic drainage includes within its meaning drainage containing organic components to be discharged in addition to organic waste water.

The present invention could solve the above described problem by the means as shown below.
(1) A method of treating organic drainage and sludge comprising an anaerobic digestion treatment step, which further comprises separating heavy metal derived compound particles in a particles-in-liquid state from two or more types of fine particles existing in the sludge of the anaerobic digestion treatment step utilizing a separation means comprising a fine particle classification means utilizing at least two properties of specific gravity, particle diameter, surface charge, magnetism and wettability of the particles.
(2) A method of treating organic drainage and sludge comprising an anaerobic digestion treatment step, which further comprises separating at least specific two types of fine particles in a particles-in-liquid state from various types of fine particles existing in the sludge of the anaerobic digestion treatment step by means of a separation means 1, and further separating the at least two types of fine particles separated by the separation means 1 into each type of fine particles, the separation means 1 and 2 each comprising a fine particle classification means utilizing at least one specific property of specific gravity, particle diameter, surface charge, magnetism and wettability of the particles.
(3) A method of treating organic drainage and sludge comprising an anaerobic digestion treatment step, which further comprises, as a process for separating at least two types of fine particles existing in sludge of the anaerobic treatment step into each type of particles from the sludge, subjecting the sludge to a foreign substance removal step by means of a sifter and a fine particle condensation step by means of a liquid cyclone, and thereafter subjecting the sludge to at least two types of steps of a fine particle separation step by flowing-film separation or a step comparable to the flowing-film separation, a fine particle separation step utilizing the difference in the sedimentation rate of the fine particles, a fine particle separation step by a jig, a fine particle separation step utilizing one or more types of sifters, and a fine particle separation step by means of a magnetic force sorter.
(4) The method of treating organic drainage and sludge according to any one of the above described items (1) to (3), wherein a crystallized substance of magnesium ammonium phosphate and heavy metal-containing particles are separated and recovered from the sludge.
(5) The method of treating organic drainage and sludge according to any one of the above described items (1) to (3), wherein the crystallized substance of magnesium ammonium phosphate and fine particles having a specific gravity of 1.3 or more other than the crystallized substance of magnesium ammonium phosphate are separated and recovered from several types of fine particles existing in the sludge of the anaerobic digestion treatment step, respectively.
(6) The method of treating organic drainage and sludge according to any one of the above described items (1) to (5), wherein the sludge of the anaerobic digestion treatment step is subjected to aeration or reduced pressure treatment before the step of separating and recovering a crystallized substance of magnesium ammonium phosphate and heavy metal-containing particles from the sludge or in the middle of the fine particle recovery process.
(7) An apparatus for treating organic drainage and sludge comprising an anaerobic digestion treatment chamber, which further comprises a separation device 1 for separating specific two or more types of fine particles in a particles-in-liquid state from various types of fine particles existing in the sludge of the anaerobic digestion treatment step and a separation device 2 for separating the two or more types of fine particles separated in the separation device 1 into each type of fine particles, wherein a fine particle classification device utilizing at least one property of specific gravity, particle diameter, surface charge, magnetism and wettability of the particles is used as each of the separation devices 1 and 2.
(8) An apparatus for treating organic drainage and sludge comprising an anaerobic digestion treatment chamber, which further comprises, as an apparatus for separating at least two types of fine particles existing in sludge of the anaerobic digestion treatment into each type of particles from the sludge, a means for subjecting the sludge to a foreign substance removal step by a sifter and a fine particle condensation step by means of a liquid cyclone, and a means for subjecting thus treated sludge to at least two types of steps of a fine particle separation step by flowing-film separation or a step comparable to the flowing-film separation, a fine particle separation step utilizing the difference in the sedimentation rate of the fine particles, a fine particle separation step by a jig, a fine particle separation step utilizing one or more types of sifters, and a fine particle separation step by means of a magnetic force sorter.

### EFFECT OF THE INVENTION

The present invention refers to a system of treating organic drainage and sludge in a sewage treatment facilities, various waste water treating facilities or the like. Particularly, the present invention refers to a process which enables comparatively easy separation and removal of heavy metal compounds existing in sludge and efficient separation and recovery of useful resources such as magnesium ammonium phosphate (hereinafter referred to as "MAP") crystals existing in the sludge at the same time. According to the present invention, recovery of valuable substances from sludge as a waste substance, removal of harmful substances and improvement of efficient utilization of discharged sludge may be accomplished at the same time.

### BRIEF DESCRIPTION OF THE DRAWINGS

Fig. 1 is a schematic explanatory diagram of the fine particle separation system of the present invention.
Fig. 2 is a flow sheet of one embodiment of process of treating organic drainage and sludge according to the present invention.
Fig. 3 is a schematic explanatory diagram of the fine particle separation system according to another embodiment of the present invention.
Fig. 4 is a flow sheet of the conventional waste water treatment system.

In the drawings, each numerals have the following meanings, respectively.
- 1: inflow water
- 2: primary sedimentation tank effluent
- 3: bioreactor effluent
- 4: treated water
- 5: primary sedimentation sludge
- 6: excess sludge
- 7: condensed sludge
- 9: digested sludge
- 10: MAP eliminated sludge
- 12: dewatered cake
- 13: filtrate from dewateration
- 14: condensation apparatus supernatant liquor
- 17: primary sedimentation tank
- 18: biological drainage treatment reactor
- 19: final sedimentation tank
- 20: sludge condensation apparatus
- 22: sludge anaerobic digestor chamber
- 23: fine particle separation system
- 25: dewatering apparatus
- 26: separated and recovered valuable substance (MAP particle and the like)
- 27: heavy metal-containing waste sludge or particles
- 28: sludge
- 29: vibration sifter
- 30: foreign substances
- 31: foreign substance eliminated digested sludge
- 32: 4 inch cyclone
- 33: fine particle condensed sludge
- 34: fine particle eliminated sludge
- 35: 2 inch cyclone
- 36: fine particle condensed sludge
- 37: fine particle eliminated sludge
- 38: discharged sludge
- 39: MGS
- 40: washing water
- 41: fine particle slurry 1
- 42: fine particle slurry 2
- 43: magnetic force sorter
- 44: magnetized product
- 100: upward washing device
- 102: washing water
- 103: fine particle eliminated sludge
- 104: fine particle slurry
- 105: fine particle slurry (harmful substance)
- 106: vibration sifter
- 107: fine particle slurry (harmful substance)

### BEST MODE FOR CARRYING OUT THE INVENTION

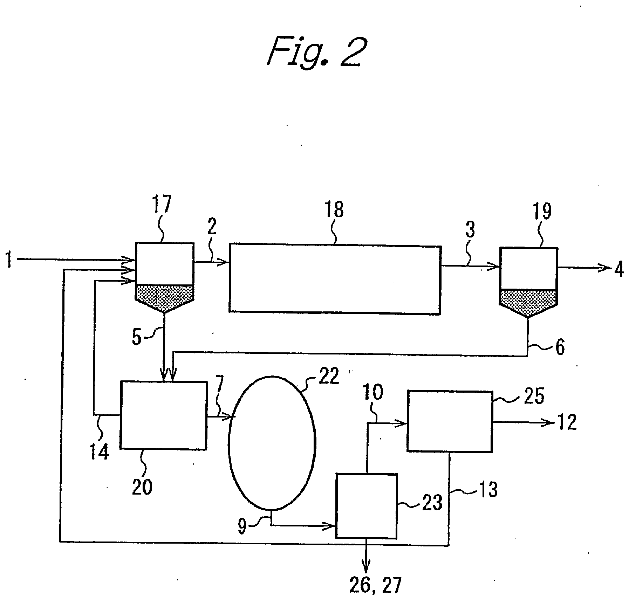

The embodiment of the present invention will be explained based on the drawings. Fig. 2 is a flow sheet for explaining one embodiment of the present invention and the present invention is not limited to this embodiment. Explanation will be made below by taking a sewage treatment facilities employing an anaerobic digestion treatment as an example of the organic drainage treatment system for explaining the embodiment of the present invention.

In Fig. 2, inflow water 1 flowing into a sewage treatment system is subjected to a biological drainage treatment, and primary sedimentation sludge 5 and excess activated sludge 6 are discharged. The sludge is condensed and separated in a sludge condensation device 20, and the condensed sludge 7 is subjected to anaerobic biological decomposition in a digestor chamber 22. As disclosed in Japanese Patent Application No. 2002-231633, MAP particles are sometimes crystallized in the digestor chamber 22 from an ammonium ion, a phosphoric ion and a magnesium ion as substrate components. Further, in order to positively form the MAP particles in the digestor chamber 22, an Mg source may be fed into the digestor chamber 22. In the digestor chamber 22, carbonic acid, hydrogen sulfide and the like are frequently formed in the course of the anaerobic decomposition of organic substances. Thus, trace amounts of heavy metals such as Cd, Cr, Mn, Pb and Fe existing in the inflow sewage mostly exist in the form of sulfides, carbonates or hydroxides.

The present inventors investigated the form of heavy metals in the anaerobic digestion sludge collected in the anaerobic digestion treatment facilities for sewer sludge which are in operation in many areas of the county. As a result, it has been found that part of the heavy metals in the sludge of a digestor chamber 22 frequently comes to particles having magnetism such as magnetic pyrite and ferrite, or is integrated with the magnetic particles (the particles are called "a group of heavy metal magnetic substance particles"), or comes to particles having a sulfide as the major component and a particle diameter of less than a couple of tens of micron meters (the particles are called "a group of heavy metal sulfide particles") or the like.

Further, it has been experimentally confirmed by the present inventors that a plurality of these types of particles existing in the sludge can be separated into each type of particles based on the differences in particle diameter, specific gravity, magnetism, surface charge and wettability of various types of particles., Further, it has also been confirmed that separability of the particles may be further increased by amplifying the differences of the mutual properties of the particles to be separated. For example, the adaptation of the addition of an iron oxide or an aluminum salt as a coprecipitator, the addition of an organic compound having a dithiocarbazide group, a thiol group, a xanthate group or the like as a sulfur compound scavenger, the ferrite method, the iron powder method, the addition of a particle surface modifier for amplifying or controlling the particle surface charge and wettability and the like, can be one effective means. Further, the anaerobic digestion sludge of the target of the present embodiment contains a large amount of a carbonic acid component in the sludge, and aeration treatment and reduced pressure treatment causes decarboxylation resulting in rise of the sludge pH. When the sludge pH raises, the MAP formation reaction, the sulfidation and hydroxylation of heavy metals are caused more easily. Accordingly, it is effective to employ such a treatment before the fine particle separation treatment or in the middle of the fine particle separation step.

According to the present embodiment, a plurality of types of fine particles which are intended to be separated/recovered by the fine particle separation system 23 can be directly separated from sludge. The digested sludge 9 containing a group of heavy metal magnetic substance particles, a group of heavy metal sulfide particles and MAP particles can be separated into MAP particles having a particle diameter of about 100 µm or more and a specific gravity of about 1.5 g/cm³ or more and heavy metal particles (the group of heavy metal magnetic substance particles, a group of heavy metal sulfide particles and the like) having a particle diameter of smaller than about a couple of tens of micrometers and a specific gravity of around about 5.0 g/cm³ and the like, respectively.

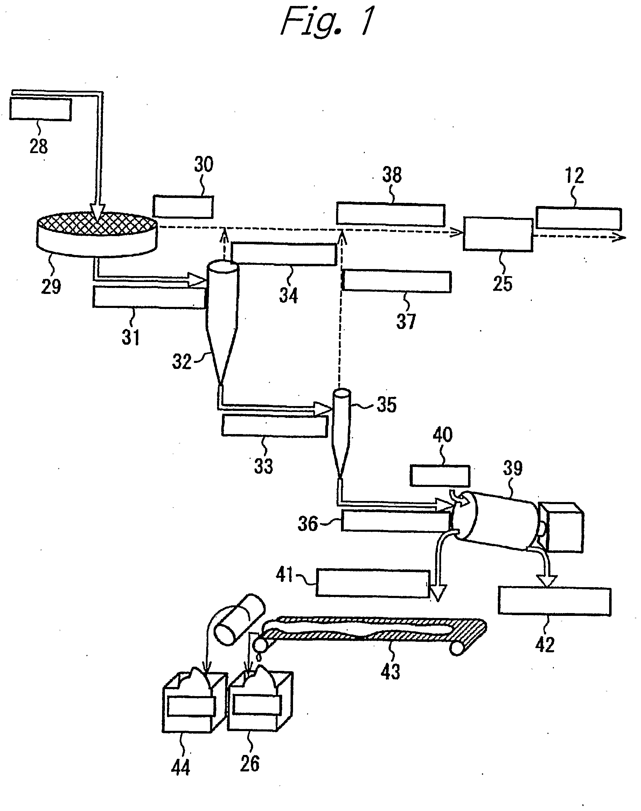

An example of the fine particle separation system 23 according to the present invention is shown in Fig. 1. The constitution of the fine particle separation system 23 to be adopted should be selected depending on heavy metal particles to be separated or the properties of a plurality of types of particles. As the present embodiment of the fine particle separation system 23, a process of systematizing a vibration sifter 29, two stages of liquid cyclones 32 and 35, a centrifugal field rocking table flowing-film separation apparatus (called "MGS" for convenience sake) 39 and a magnetic force sorter 43 in series was employed. MGS is a trade name of a flowing-film separation apparatus manufactured by Mozley (GB) and abbreviation of "Multi Gravity Separator". In Fig. 1, the wet type magnetic force sorter 43 is shown, but dry type magnetic force sorter may also be used. First, the sludge 28 which is supplied to the present fine particle separation system 23 enters into the vibration sifter 29, where foreign substances 30 such as hairs and seeds of plants are removed from the sludge. After removal of fibrous substances and particles having a particle diameter of a couple of millimeters or more, heavy metal particles and MAP particles are simultaneously condensed to about 20 to 400 times by the two-stage liquid cyclones 32 and 35. In this instance, part of the group of heavy metal sulfide particles having a comparatively small particle diameter sometimes overflows from the overflow side of the second stage liquid cyclone 35. Thus, if necessary, it is possible to supply the overflowed liquor to MGS 39 to separate the group of heavy metal sulfide particles. If necessary, the fine particle condensation step by a liquid cyclone may be performed in one stage or a plurality of stages.

Discharged sludge 38 in Fig. 1 corresponds to MAP eliminated sludge 10 in Fig. 2. Magnetized substances 44 in Fig. 1 corresponds to heavy metal containing sludge or particles 27 in Fig. 2.

There are a plurality of choices for the method of treating the sludge 36 containing the condensed heavy metal particles and MAP particles to thereby separate the two types of particles from the sludge, depending on various treatment environments. Some specific examples will be shown below.
(First Embodiment) For example, when there is a restriction to the amount of washing water which can be used in the fine particle separation process in sludge treatment facilities and only less than 5% of washing water based on the amount of the target sludge can be used, the sludge 36 is supplied to the MGS 39 and separated into heavy metal particles and the MAP particles by the difference in the particle diameter and the specific gravity of particles. The MGS 39 may be expressed as an apparatus obtained by rolling up the horizontal plane of a general-purpose rocking table and packing the resulting roll into a rotary drum, and it can exert a gravitational force of a couple of times the normal one on particles. This allows MGS 39 to sort finer particles as well than the general-purpose table. The MGS 39 is an apparatus capable of separating particles depending on particle diameter and specific gravity by controlling the number of revolutions of the drum and the washing ratio. The MGS mostly has two or more discharge outlets for particles. The method of the present invention has employed a method of obtaining two types of groups of (1) a group of high specific gravity particles and (2) a group of low specific gravity particles and slime particles. As a result of investigating and optimizing various operational conditions of the MGS, it has succeeded to find operational conditions by which the ratio of MAP particles and that of heavy metal particles in the group of high specific density particles are made about 70% and about 20%, respectively, and the ratio of the MAP particles and that of heavy metal particles in the group of low specific density particles and slime particles are made about 3% and about 10 to 40%, respectively.

In thus obtained group of low specific gravity particles and slime particles, the ratio of the MAP particles as a valuable substance is low and that of the heavy metal particles is high. Thus, the sludge 42 as such is separated as heavy metal-containing waste sludge. The group of high specific gravity particles 41 is introduced, as a product from MGS, to a magnetic force sorter 43 of the next step. Further, it has been found by the investigations heretofore that, both the group of heavy metal magnetic substance particles and the group of heavy metal sulfide particles frequently exist in the group of low specific gravity particles and slime particles. Also, it has been found that the heavy metal magnetic substance particles are separated in the magnetic force sorter 43, and particularly most particles containing a sulfur component are very efficiently separated as a magnetized product from MAP particles. In general, most heavy metal sulfides have no magnetism but it may be surmised that the heavy metal sulfides come to particles having magnetism in the state of the heavy metals mixed with a sulfur component like a magnetic pyrite or the like. However, the details of the mechanism of this phenomenon have not been clarified.

As a result, in some cases, the ratio of the MAP particles in the recovered particles 26 after magnetic force sorting comes to about 90% or more and the ratio of the heavy metal particles comes to less than 1%. If necessary, the liquor separated in respective unit separation devices and the particles in the liquor may be further supplied to another separation device to increase the recovery ratio, removal ratio of particles and the like and meet the target ratio.. For example, in some cases, MAP particles and heavy metal fine particles or a liquor containing them may be supplied to a sifter having an opening diameter of 100 to 250 µm in the former stage or the latter stage of magnetic force sorting in the separation operation of these two groups of particles in the anaerobic digestion sludge. In this case, the MAP particles can be separated on the upper side of the sifter while the heavy metal fine particles can pass through the sifter to be separated downwardly. Furthermore, by adopting the present system, the amount of washing water used frequently comes to less then 5% based on the amount of the target sludge. The constitution of the present fine particle separation system 23 is an improved system of the MAP separation system disclosed in "Method and Apparatus for treating Organic Waste Water and Sludge": Japanese Patent Application No. 2002-328336.

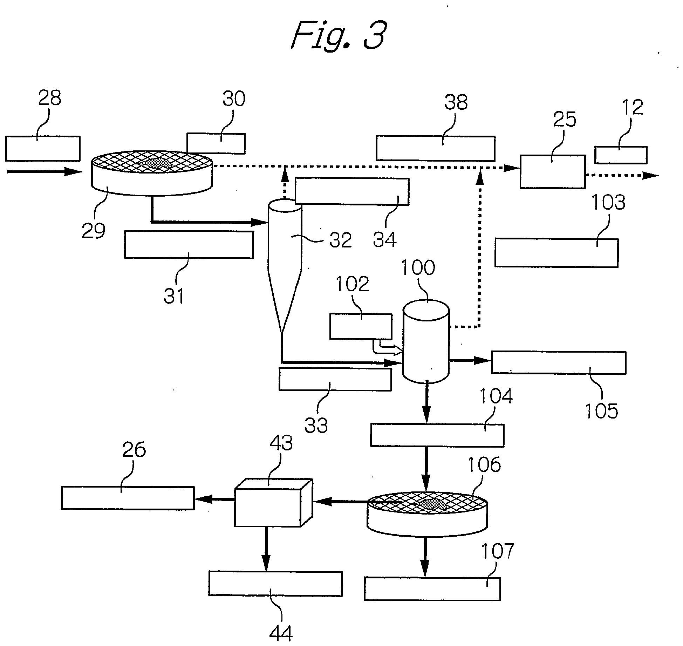

The foreign substances 30 separated from the condensed sludge 7 by the fine particle separation system 23 and the liquid cyclone overflow sludge 34, 37 has a small ratio of both heavy metal particles and MAP particles. Accordingly, these may be mixed and subjected to weight reduction treatment such as dewatering treatment, and thereafter may be subjected to waste material treatment or may be utilized as a biomass effective resource. The smaller ratios of the heavy metals and phosphorus derived from the MAP, are the more advantageous for making the sludge into compost or a raw material of cement.
(Second Embodiment) Another embodiment of the present invention is shown in a flow sheet of Fig. 3. For example, when a restricted amount of washing water which can be used is less than 10% of the amount of the target sludge in the fine particle separation process, the sludge 33 discharged from the 4-inch cyclone 32 is introduced to an upflow washing device 100 to wash the sludge with upflow washing water 102 in an amount of about 2 to 10 times the amount of the sludge 36. In the upflow washing device 100, a plurality of types of particles are separated by utilizing the difference in the sedimentation rate of each type of particles and simultaneously washed. By setting the linear velocity (LV) of a mixed liquor of the sludge and the washing water at a predetermined velocity, for example, 20 to 50 m/h, fine particles with different specific gravities and particles diameters are separated in the form of respective layers. In this instance, particles with a small specific gravity and a small diameter have a sedimentation rate lower than the predetermined linear velocity. Accordingly, they cannot stay in the upflow washing device 100, and are washed away. Each type of particles which are retained in the device to form a separate layer in accordance with their sedimentation rate can be withdrawn by a withdrawal tube having a different height provided for the device 100, and thus can be separated and recovered as particle slurries 104 and 105. In Fig. 3, particle slurry 105 is discharged as harmful substance. For example, at a linear velocity (LV) of 40 m/h, the MAP particles swells twice while some heavy metal particles sometimes swell 4 to 7 times. However, when one type of particles with a large specific gravity and a small particle diameter and another type of particles with a small specific gravity and a large particle diameter have nearly the same sedimentation rate, these types of particles cannot be separated by the upflow washing device 100 alone in some cases. In this instance, it is effective to use a dry or wet separation means and separate the particle utilizing the difference in the particle diameter of each types of fine particle by a sifter as the latter stage treatment process of the upflow washing device 100. The MAP particles can be separated from the heavy metal particles as particle slurry 107 to some extent by a sifter 106 having an opening diameter of 100 to 250 µm. When one type of particles has magnetism, it is effective to separate each type of particles by a dry or wet magnetic force sorter 43. The MAP particles and the magnetic substance particles or magnetic substance-attached particles can be efficiently separated at about 10,000 to 20,000 G as non-magnetized substance 26 and magnetized substance 44, respectively. In some cases, a fine particle classification step by pulsation such as a pneumatic jig is also effective as a substitute machine for the upflow washing device. As an example of adopting the case of this second embodiment in the separation operation of the MAP particles and the heavy metal fine particles in sewage anaerobic digestion sludge, by supplying the groups of particles condensed by a liquid cyclone to a magnetic force sifter having an opening diameter of 100 to 250 µm in the latter or former stage, the MAP particles may be separated on the upper side of the sifter while the heavy metal fine particles may pass through the sifter to be separated downwardly.

As explained above, by employing the present process which can separate MAP particles and heavy metal particles in the anaerobic digestion sludge, respectively, it is possible to separate and remove heavy metal particles of harmful substances and to effectively recover MAP particles of a valuable substance.

### Example

Next, one example of the operational results of an experimental plant into which the waste water treatment technique of the present invention is actually incorporated will be explained but the present invention is not limited to this example.

The present working example was a pilot plant experiment carried out by using the anaerobic digestion sludge in a sewage treatment facilities A and the flow is the same as the flow of the previously shown Fig. 2. The object is to carry out recovery of MAP particles as a phosphorus resource existing in sludge and removal of harmful particles such as heavy metal particles in the sludge to improve the utilization efficiency of the discharged sludge as a raw material of cement. The sewage treatment facilities A employs activated sludge treatment by the anaerobic and aerobic method. In the anaerobic digestor chamber 22, 35°C middle temperature digestion is performed and the dwell time is 25 days.

In the working example, as the step of fine particle separation 23, procedure shown in Fig. 1 was conducted. Specifically, sludge 28 was introduced to vibration shifter 29 having pore size of 1.2 mm to remove foreign substances 30. The slurry was then introduced to 4-inch cyclone 32 and then 2-inch cyclone 35 in series to concentrate fine particles in the sludge to about 180 times to thereby obtain fine particle concentrated sludge 36. Then the slurry was introduced to MGS 39 operated in a condition of set angle of 4°, revolution speed of 300 rpm and washing water amount of 1.5 times the sludge to wash and separate sludge. Fine particle slurry 41 which was mixture of MAP particles and heavy metal containing particles was drained and then dried at 40°C and thereafter subjected to dry type magnetic force sorter 43 of 10,000 gauss to separate magnetized substances 44 and non-magnetized substances 26. MAP particles were collected as non-magnetized substances 26, and heavy metal containing particles were separated as magnetized particles 44.

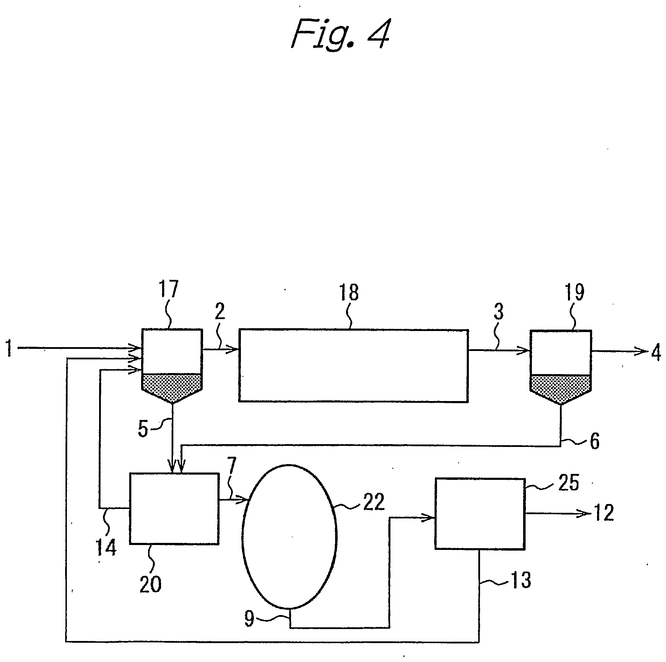

As a comparative system to the present working example, two treatment systems of a conventional process of not performing MAP recovery and heavy metal treatment as shown in Fig. 4, and a process of separating and recovering MAP particles alone from anaerobic digestion sludge which was described in Japanese Patent Application No. 2002-328336, were performed in parallel. The process of Japanese Patent Application No. 2002-328336 was the same as the process of the working example as mentioned above except that separation by means of magnetic force sorter 43 was not used and sludge was concentrated by means of 2-step cyclones 32 and 35 to 260 times, and MGS was operated at revolution speed of 220 rpm.

In these three systems, the same digested sludge was used. The results of operating sludge treatment are shown in Table 1. The numerical values in the Table are all average values of the treatment results for an operation period of about one month.

**Table 1 Results of Operating Sludge Treatment**

| | Present Invention | method described in 2000-328336 | Conventional process |
|---|---|---|---|
| Digested Sludge SS (g/L) | 25.1 | 25.1 | 25.1 |
| Digested Sludge MAP Content (g/L) | 0.82 | 0.82 | 0.82 |
| MAP Recovery Ratio^{*1} | 92.1 | 85.4 | 0 |
| Discharged Sludge SS (g/L) | 21.8 | 23.1 | 24.7 |
| Fe Ratio in Discharged Sludge^{*2} (mg/kg) | 39,500 | 42,100 | 42,400 |
| Mn Ratio in Discharged Sludge^{*2} (mg/kg) | 2,100 | 2,690 | 2,740 |
| Zn Ratio in Discharged Sludge^{*2} (mg/kg) | 990 | 1,490 | 1,530 |
| Ti Ratio in Discharged Sludge^{*2} (mg/kg) | 420 | 750 | 760 |
| Fe Ratio in Heavy Metal Waste Sludge^{*3} (%) | 31.1 | - | - |
| Mn Ratio in Heavy Metal Waste Sludge^{*3} (%) | 5.2 | - | - |
| Zn Ratio in Heavy Metal Waste Sludge^{*3} (%) | 4.4 | - | - |
| Ti Ratio in Heavy Metal Waste Sludge^{*3} (%) | 2.9 | - | |

| | | | |
|---|---|---|---|
| *1 is a ratio of the MAP recovered from the MAP separation condensation machine to the MAP content in the digested sludge. *2 is a ratio of Fe, Mn, Zn or Ti in the inorganic component of the discharged sludge. *3 is a ratio of Fe, Mn, Zn or Ti in the inorganic component of the heavy metal waste sludge. | | | |

The amount of suspended solids (SS) in the digested sludge used in the example was 25.1 g/L and the amount of MAP in the sludge was 0.82 g/L. The recovery ratio of MAP was 92.1% according to the present invention while the recovery ratio of MAP is 0% according to the conventional example and 85.4% according to the method of 2000-328336. The recovery ratio of MAP according to the present invention became higher than that according to the method of 2000-328336 by 6.7 points. The result by the present invention is remarkably higher than the method of 2002-228336 with respect to the recovery ratio of MAP. This might be for the following reason. In the method of 2002-328336, the target particles to be separated and recovered were only MAP particles having good separability of a median diameter of about 500 µm, and thus a liquid cyclone having a comparatively high concentration ratio was employed. To the contrary, in the method of the present invention, in order to simultaneously separate MAP particles having a comparatively large diameter and a comparatively low specific density and heavy metal particles having a comparatively small diameter and a comparatively high specific density, a liquid cyclone having a comparatively low condensation ratio was used so as to recover particles in a somewhat broader range of size. Further, in the method of the present invention, more operational factors than the method of 2002-328336 were used, including setting of the dilution ratio and the number of revolutions in MGS, the slurry supply speed to the magnetic force sorter, the belt speed, the strength of magnetic force and the like. Accordingly, the fine particle separation system 23 according to the present invention can be highly optimized.

The amount of SS in the discharged sludge was 21.8 g/L according to the present invention while it was 24.7 g/L according to the conventional method and 23.1 g/L according to the method of 2002-328336. The amount of the discharged SS according to the method of 2002-328336 is smaller than that according to the conventional method, because the MAP and foreign substances were separated from the sludge in the MAP recovery process. The amount of SS according to the present invention is further smaller. This is because heavy metal waste sludge was further eliminated from the sludge. The heavy metal ratio in the discharged sludge became smaller in the order of the conventional method = method of 2002-328336 > the present invention. It may be said that the heavy metal ratios in the discharged sludge of the conventional method and method of 2002-328336 were at the same level and the heavy metal ratio in the discharged sludge of the present invention was greatly reduced compared to that of the conventional method and method of 2002-328336. When each heavy metal ratio in the discharged sludge according to the conventional method is taken as 100%, the reduction of Fe came to about 7%; that of Mn came to about 23%; that of Zn came to 35%; and that of Ti came 45%, according to the present invention. Thus, each heavy metal ratio in the discharged sludge was reduced according to the present invention. That is, by employing the system of the present invention, the heavy metal content in the discharged sludge could be remarkably reduced. Particularly with respect to Mn, Zn and Ti, a reduction in removal ratio of 23% to 45% could be attained. Thus, when the discharged sludge is subjected to weight reduction treatment such as dewatering treatment and utilized as a compost or a raw material of cement, the sludge can be recycled as sludge which hardly causes heavy metal hindrance and the like and is easily handled. The waste sludge containing a small amount of heavy metals separated in the method of the present invention requires an adequate industrial waste treatment.

The process of the present invention enables relatively easy separation and removal of heavy metal compounds existing in sludge and efficient separation and removal of useful resources such as magnesium ammonium phosphate (MAP) crystals in the sludge at the same time. According to the method of the present invention, the recovery of a valuable substance from sludge as a waste material, the removal of harmful substances and the improvement of effective utilization efficiency of discharged sludge can be simultaneously accomplished. Thus, the present invention provides a useful technique which can be broadly employed as a treating method and a treating apparatus for organic drainage and sludge containing high concentration organic substances, phosphorus, nitrogen and a small amount of heavy metals in a sewage treatment facilities and various waste water treatment facilities.

## Claims

1. A method of treating organic drainage and sludge comprising an anaerobic digestion treatment step, comprising:
feeding an Mg source into a digested sludge resulted from the anaerobic digestion treatment step, thereby forming a crystallized substance of magnesium ammonium phosphate,
separating the crystallized substance of magnesium ammonium phosphate and heavy metal-containing particles from the digested sludge by subjecting the digested sludge to a foreign substance removal step by means of a sifter and subsequently to a fine particle condensation step by means of a liquid cyclone, and
recovering the crystallized substance of magnesium ammonium phosphate and the heavy metal-containing particles separately by subjecting the sludge to at least two types of steps of a fine particle separation step by flowing-film separation, a fine particle separation step utilizing the difference in the sedimentation rate of the fine particles, a fine particle separation step by a jig, a fine particle separation step utilizing one or more types of sifters, and a fine particle separation step by means of a magnetic force sorter.

2. The method of treating organic drainage and sludge according to claim 1, wherein the crystallized substance of magnesium ammonium phosphate and fine particles having a specific gravity of 1.3 or more other than the crystallized substance of magnesium ammonium phosphate are separated and recovered from several types of fine particles existing in the sludge of the anaerobic digestion treatment step, respectively.

3. The method of treating organic drainage and sludge according to Claim 1, **characterized in that** the digested sludge resulted from the anaerobic digestion treatment step is subjected to aeration or reduced pressure treatment before the step of separating and recovering a crystallized substance of magnesium ammonium phosphate and heavy metal-containing particles from the sludge or in the middle of the fine particle recovery process.

4. An apparatus for treating organic drainage and sludge comprising an anaerobic digestion treatment chamber, comprising:
a means for feeding an Mg source into a digested sludge resulted from an anaerobic digestion treatment step,
a separation device 1 for separating a crystallized substance of magnesium ammonium phosphate and heavy metal-containing particles in a particles-in-liquid state from various types of fine particles existing in the digested sludge and
a separation device 2 for separately recovering the crystallized substance of magnesium ammonium phosphate and the heavy metal-containing particles,
wherein a fine particle classification device utilizing at least one property of specific gravity, particle diameter, surface charge, magnetism and wettability of the particles is used as each of the separation devices 1 and 2, and
wherein, the separation device 1 comprises a sifter for subjecting the digested sludge to foreign substance removal and a liquid cyclone for carrying out fine particle condensation, and that the separation device 2 is selected from the group consisting of a flowing-film separator, a fine particle separator utilizing differences in the sedimentation rate of the fine particles, a jig, a sifter and a magnetic force sorter.

## Patentansprüche

1. Verfahren zur Behandlung von organischer Drainage und Schlamm, das einen anaeroben Faulungsbehandlungsschritt aufweist, der Folgendes aufweist:
Zuführen einer Mg-Quelle zu einem Faulschlamm, was zu einem anaeroben Faulungsbehandlungsschritt führt, wodurch eine kristallisierte Substanz aus Magnesiumammoniumphosphat gebildet wird,
Trennen der kristallisierten Substanz aus Magnesiumammoniumphosphat und Schwermetall enthaltenden Partikeln aus dem Faulschlamm, indem der Faulschlamm einem Fremdkörperentfernungsschritt mittels eines Siebs und nachfolgend einem Feinpartikelkondensationsschritt mittels eines Flüssigkeitsfliehkraftabscheiders unterzogen wird, und
Rückgewinnen der kristallisierten Substanz des Magnesiumammoniumphosphats und der Schwermetall enthaltenden Partikel in separater Weise, indem der Schlamm zumindest zwei Arten von Schritten eines Feinpartikelabtrennungsschritts durch Flussfilmtrennung, eines Feinpartikelabtrennungsschritts durch Nutzung der Differenz in der Sedimentierungsrate der Feinpartikel, eines Feinpartikelabtrennungsschritts durch eine Absetzvorrichtung, eines Feinpartikelabtrennungsschritt durch Nutzen von einem oder mehreren Arten von Sieben, und eines Feinpartikelabtrennungsschritt mittels einer Magnetkraftsortierungsvorrichtung unterzogen wird.

2. Verfahren zur Behandlung von organischer Drainage und Schlamm gemäß Anspruch 1, wobei die kristallisierte Substanz aus Magnesiumammoniumphosphat und die Feinpartikel, die eine spezifische Gravität bzw. Schwere von 1,3 oder mehr anders als die kristallisierte Substanz von Magnesiumammoniumphosphat haben, getrennt werden und aus den mehreren Arten von Feinpartikeln rückgewonnen werden, die jeweils in dem Schlamm des anaeroben Faulungsbehandlungsschritts existieren.

3. Verfahren zur Behandlung von organischer Drainage und Schlamm gemäß Anspruch 1, **dadurch gekennzeichnet, dass** der Faulschlamm, der aus dem anaeroben Faulungsschritt resultiert, einer Durchlüftung oder Behandlung mit reduziertem Druck vor dem Schritt des Trennens und Rückgewinnens einer kristallisierten Substanz aus Magnesiumammoniumphosphat und Schwermetall enthaltenden Partikeln aus dem Schlamm oder in der Mitte des Feinpartikelrückgewinnungsprozesses unterzogen wird.

4. Vorrichtung zur Behandlung von organischer Drainage und Schlamm, die eine anaerobe Faulbehandlungskammer aufweist, die Folgendes aufweist:
Mittel zum Zuführen einer Mg-Quelle in einen Faulschlamm, der aus einem anaeroben Faulbehandlungsschritt resultiert,
eine Trennvorrichtung 1 zur Trennung einer kristallisierten Substanz aus Magnesiumammoniumphosphat und Schwermetall enthaltenden Partikeln in einem Partikel-in-Flüssigkeit-Zustand aus verschiedenen Arten von Feinpartikeln, die in dem Faulschlamm existieren, und
eine Trennvorrichtung 2 zur separaten Rückgewinnung der kristallisierten Substanz aus Magnesiumammoniumphosphat und den Schwermetall enthaltenden Partikeln,
wobei eine Feinpartikelklassifizierungsvorrichtung, die zumindest eine Eigenschaft aus spezifischer Schwere, Partikeldurchmesser, Oberflächenladung, Magnetismus und Benetzbarkeit der Partikel nutzt, als jede der Trennvorrichtungen 1 und 2 verwendet wird, und
wobei die Trennvorrichtung 1 ein Sieb aufweist, um den Faulschlamm einer Fremdkörperentfernung zu unterziehen und einen Flüssigkeitsfliehkraftabscheider um die Feinpartikelkondensation auszuführen, und wobei die Trennvorrichtung 2 aus der Gruppe ausgewählt wird, die aus einer Flussfilmtrennvorrichtung, eine Feinpartikeltrennvorrichtung, die Unterschiede in der Sedimentierungsrate der Feinpartikel nutzt, einer Setzvorrichtung, einem Sieb und einer Magnetkraftsortiervorrichtung besteht.

## Revendications

1. Procédé de traitement de drainage organique et de boue comprenant une étape de traitement par digestion anaérobie, comprenant :
fournir une source de Mg dans une boue digérée résultant de l'étape de traitement par digestion anaérobie, pour former ainsi une substance cristallisée de phosphate de magnésium et d'ammonium,
séparer la substance cristallisée de phosphate de magnésium et d'ammonium et des particules lourdes contenant du métal à partir de la boue digérée en soumettant la boue digérée à une étape de retrait de substances étrangères au moyen d'un tamis et ensuite à une étape de condensation de particules fines au moyen d'un cyclone liquide, et
récupérer séparément la substance cristallisée de phosphate de magnésium et d'ammonium et les particules lourdes contenant du métal en soumettant la boue à au moins deux types d'étapes constituées d'une étape de séparation de particules fines par une séparation à film de circulation, d'une étape de séparation de particules fines utilisant la différence de vitesse de sédimentation des particules fines, d'une étape de séparation de particules fines dans un bac, d'une étape de séparation de particules fines utilisant un ou plusieurs types de tamis, et d'une étape de séparation de particules fines au moyen d'une trieuse par force magnétique.

2. Procédé de traitement de drainage organique et de boue selon la revendication 1, dans lequel la substance cristallisée de phosphate de magnésium et d'ammonium et les particules fines ayant une densité de 1,3 ou plus autres que la substance cristallisée de phosphate de magnésium et d'ammonium sont séparées et récupérées à partir de plusieurs types de particules fines existant dans la boue de l'étape de traitement par digestion anaérobie, respectivement.

3. Procédé de traitement de drainage organique et de boue selon la revendication 1, **caractérisé en ce que** la boue digérée obtenue par l'étape de traitement par digestion anaérobie est soumise à une aération ou un traitement à pression réduite avant l'étape de séparation et de récupération d'une substance cristallisée de phosphate de magnésium et d'ammonium et de particules lourdes contenant du métal à partir de la boue ou au milieu du processus de récupération de particules fines.

4. Dispositif de traitement de drainage organique et de boue, comprenant une chambre de traitement par digestion anaérobie, comprenant :
des moyens pour fournir une source de Mg dans une boue digérée résultant d'une étape de traitement par digestion anaérobie,
un dispositif de séparation 1 pour séparer une substance cristallisée de phosphate de magnésium et d'ammonium et des particules lourdes contenant du métal dans un état de particules dans un liquide, à partir de divers types de particules fines existants dans la boue digérée, et
un dispositif de séparation 2 pour récupérer séparément la substance cristallisée de phosphate de magnésium et d'ammonium et les particules lourdes contenant du métal,
dans lequel un dispositif de classification de particules fines utilisant au moins une propriété parmi la densité, le diamètre de particule, la charge surfacique, le magnétisme et la mouillabilité des particules est utilisé pour chacun des dispositifs de séparation 1 et 2, et
dans lequel, le dispositif de séparation 1 comprend un tamis pour soumettre la boue digérée à un retrait de substances étrangères et un cyclone liquide pour réaliser une condensation de particules fines, et le dispositif de séparation 2 est choisi dans le groupe constitué d'un séparateur à film de circulation, d'un séparateur de particules fines utilisant des différences de vitesse de sédimentation des particules fines, un bac, un tamis et une trieuse par force magnétique.
